(19) Europäisches Patentamt
European Patent Office
Office européen des brevets

(11) EP 3 539 474 A1

(12) **EUROPÄISCHE PATENTANMELDUNG**

(43) Veröffentlichungstag:
18.09.2019  Patentblatt 2019/38

(51) Int Cl.:
*A61B 6/00* (2006.01)

(21) Anmeldenummer: 18161773.9

(22) Anmeldetag: 14.03.2018

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**
Benannte Erstreckungsstaaten:
**BA ME**
Benannte Validierungsstaaten:
**KH MA MD TN**

(71) Anmelder: **Siemens Healthcare GmbH**
**91052 Erlangen (DE)**

(72) Erfinder: **Dressler, Christian**
**91074 Herzogenaurach (DE)**

(54) **VERFAHREN ZUR AUTOMATISCHEN POSITIONIERUNG EINES AUFNAHMESYSTEMS UND RÖNTGENBILDGEBUNGSSYSTEM**

(57)    Die Erfindung umfasst ein Verfahren zur automatischen Positionierung eines Aufnahmesystems mit einem Röntgendetektor und einer Röntgenquelle bezüglich eines Teilbereichs eines Untersuchungsobjekts, mit den folgenden Schritten:

• Erste Bilderfassung des Untersuchungsobjekts,

• Entgegennahme einer Benutzereingabe hinsichtlich eines interessierenden Teilbereichs des Untersuchungsobjekts auf dem ersten Bild und Speicherung der Position des Teilbereichs;

• Verstellung des Aufnahmesystems derart, dass eine hubfreie Verschiebung zwischen Aufnahmesystem und Untersuchungsobjekt in einer Ebene parallel zum Röntgendetektor resultiert,

• Speicherung des Verschiebeabstands ($\Delta$m),

• Zweite Bilderfassung des Untersuchungsobjekts und Bestimmung der Position des interessierenden Teilbereichs auf dem zweiten Bild,

• Bestimmung des relativen Pixelabstands ($\Delta$d) zwischen der Position des interessierenden Teilbereichs auf dem ersten und auf dem zweiten Bild;

• Berechnung des SOD (Röntgenquelle-Untersuchungsobjekt-Abstands) aus dem Verschiebeabstand, dem relativen Pixelabstand und dem bekannten SDD (Röntgenquelle-Röntgendetektor-Abstand), und

• Verwendung des SOD für eine automatische Positionierung des Aufnahmesystems

FIG 1

**Beschreibung**

Die Erfindung betrifft ein Verfahren zur automatischen Positionierung eines Aufnahmesystems mit einem Röntgendetektor und einer Röntgenquelle bezüglich eines Teilbereichs eines Untersuchungsobjekts gemäß dem Patentanspruch 1 sowie ein Röntgenbildgebungssystem gemäß dem Patentanspruch 7.

Durch die Kegelform der Röntgenstrahlung ist die Größe eines Objektes auf dem Röntgenbild abhängig von der Entfernung des Objekts zum Röntgendetektor und zur Röntgenquelle. Um einen bestimmten Punkt oder Teilbereich eines Patienten bzw. Untersuchungsobjekts, z.B. eines Wirbelkörpers eines Patienten, auf dem Bild mit einem Aufnahmesystem exakt anzufahren, d.h. zu zentrieren, ist es notwendig, den genauen Abstand zwischen der Röntgenquelle und dem Objekt (SOD) zu kennen. Das Aufnahmesystem kann z.B. von einem C-Bogen gebildet werden, an welchem eine Röntgenquelle und ein Röntgendetektor befestigt sind. Ist der SOD unbekannt, so kann der entsprechende Punkt oder Teilbereich nicht zentriert werden.

Bisher wurde für den SOD häufig ein Durchschnittswert angenommen und der Bewegungsfehler in Kauf genommen, so dass eine Zentrierung eines bestimmten Punktes oder Teilbereichs sehr ungenau erfolgt. Ultraschallsensoren oder Time-of-Flight-Sensoren messen i.A. nur den Abstand der Röntgenquelle zum Patientenkörper als solchem, nicht aber zum gewünschten Objekt oder Punkt im Patientenkörper, also z.B. einem Wirbelkörper. In speziellen Fällen wird mit standardisierten Referenzobjekten, z. B. Kugeln, gearbeitet, deren Größe dem System bekannt ist, und daraus Abstände auf einem Röntgenbild bestimmt.

Es ist Aufgabe der vorliegenden Erfindung, ein Verfahren zur Positionierung eines Aufnahmesystems mit einem Röntgendetektor und einer Röntgenquelle bezüglich eines Teilbereichs eines Patienten bereitzustellen, welches eine besonders exakte, automatische Positionierung von Teilbereichen ermöglicht; des Weiteren ist es Aufgabe der Erfindung, ein für die Durchführung des Verfahrens geeignetes Röntgenbildgebungssystem bereitzustellen.

Die Aufgabe wird erfindungsgemäß gelöst durch ein Verfahren zur automatischen Positionierung eines Aufnahmesystems mit einem Röntgendetektor und einer Röntgenquelle bezüglich eines Teilbereichs eines Untersuchungsobjekts gemäß dem Patentanspruch 1 und von einem Röntgenbildgebungssystem gemäß dem Patentanspruch 7. Vorteilhafte Ausgestaltungen der Erfindung sind jeweils Gegenstand der zugehörigen Unteransprüche.

Bei dem erfindungsgemäßen Verfahren zur automatischen Positionierung eines Aufnahmesystems mit einem Röntgendetektor und einer Röntgenquelle bezüglich eines Teilbereichs eines Untersuchungsobjekts werden eine erste Bilderfassung des Untersuchungsobjekts, eine Entgegennahme einer Benutzereingabe hinsichtlich eines interessierenden Teilbereichs des Untersuchungsobjekts auf dem ersten Bild und Speicherung der Position des Teilbereichs, eine automatische Verstellung des Aufnahmesystems derart, dass eine insbesondere hubfreie Verschiebung zwischen Aufnahmesystem und Untersuchungsobjekt in einer Ebene parallel zum Röntgendetektor resultiert, eine Speicherung des Verschiebeabstands ($\Delta$m), eine zweite Bilderfassung des Untersuchungsobjekts und Bestimmung der Position des interessierenden Teilbereichs auf dem zweiten Bild, eine Bestimmung des relativen Pixelabstands ($\Delta$d) zwischen der Position des interessierenden Teilbereichs auf dem ersten und auf dem zweiten Bild, und eine Berechnung des Röntgenquelle-Teilbereich-Abstands (SOD) aus dem Verschiebeabstand, dem relativen Pixelabstand und dem bekannten Röntgenquelle-Röntgendetektor-Abstand (SDD) durchgeführt, und anschließend der SOD für eine automatische Positionierung des Aufnahmesystems, insbesondere eine Zentrierung des Teilbereichs bezüglich des Röntgendetektors, verwendet.

Unter einem Teilbereich des Untersuchungsobjekts wird dabei ein anatomisches Objekt oder ein Ausschnitt aus dem Untersuchungsobjekt oder ein Markerpunkt verstanden, welche maximal die Hälfte, insbesondere weniger, eines Röntgenbildes ausfüllen. Beispiele hierfür sind Wirbelkörper oder Knochen oder kleine Organe oder markante Hohlorgane. Die Benutzereingabe kann derart durchgeführt werden, dass ein Nutzer das erste Bild angezeigt bekommt und darauf den Teilbereich markiert, z.B. mittels Mausklick oder über einen Touchscreen oder auf einem Smart Device. Die entsprechende Position des Teilbereichs wird dann gespeichert. Unter einer hubfreien Verschiebung wird eine Verschiebung in einer Ebene parallel zum Röntgendetektor ohne einen Hub bezüglich des Röntgendetektors/der Röntgenquelle verstanden, so dass der SOD während der Verschiebung nicht verändert wird. Sollte sich der Hub verändern, so muss dies in die Berechnungen aufgenommen werden. Die Verstellung des Aufnahmesystems mit resultierender Verschiebung zwischen Aufnahmesystem und Untersuchungsobjekt in einer Ebene parallel zum Röntgendetektor kann automatisch angesteuert werden, z.B. von einer das Aufnahmesystem ansteuernden Systemsteuerung. Bevorzugt wird dabei der Teilbereich an das Zentrum des Röntgendetektors angenähert. Dies kann z.B. unter Verwendung von einem Durchschnittswert für den SOD wie aus dem Stand der Technik bekannt durchgeführt werden.

Der Verschiebeabstand kann dabei im Allgemeinen von dem Aufnahmesystem bzw. der dieses steuernden Systemsteuerung ausgegeben werden. Zur Bestimmung des Pixelabstands kann z.B. vom Zentrum des Teilbereichs oder einem anderen bestimmten Punkt des Teilbereichs ausgegangen werden und dieser auf den beiden Bildern miteinander verglichen werden.

Das zugrundeliegende Problem, dass eine genaue Positionierung bzw. sogar Zentrierung eines Teilbereichs eines Untersuchungsobjekts sehr schwierig ist und zu ungenauer Positionierung des Teilbereichs führt, wird dadurch gelöst, dass zunächst automatisch der exakte Röntgenquelle-Teilbereich-Abstand bestimmt wird. Ist der SOD bekannt, so kann auf einfache Weise eine genaue Positionierung berechnet und ausgeführt werden. Das Verfahren kann auf sehr einfache Weise ohne zusätzliche Referenzobjekte während des normalen klinischen Ablaufs und sogar während eines Eingriffs am Patienten durchgeführt werden. Das Ergebnis ist sehr genau und erlaubt sehr präzise Positionierungen. Das Verfahren kann für den Benutzer in einem sehr einfachen Workflow umgesetzt werden, in dem er lediglich den gewünschten Teilbereich bzw. einen Punkt darin markiert. Im Anschluss an das Verfahren können auch andere, sich in dem Untersuchungsobjekt in derselben Ebene wie der Teilbereich befindliche weitere Teilbereiche präzise bezüglich des Röntgendetektors positioniert werden. Im Falle z.B. eines Wirbelkörpers als Teilbereich können auch angrenzende Wirbelkörper präzise positioniert werden.

Insbesondere wird der SOD nach dem Strahlensatz, also der Formel $SOD = SDD \cdot \frac{\Delta m}{\Delta d}$ berechnet.

Nach einer Ausgestaltung der Erfindung wird die Position des interessierenden Teilbereichs auf dem zweiten Bild automatisch mittels eines Algorithmus zur Bilderkennung oder Kantendetektion oder Objekterkennung ermittelt. Hierdurch kann das Verfahren nahezu komplett automatisiert und dadurch sehr schnell und fehlerfrei ablaufen.

Nach einer weiteren Ausgestaltung der Erfindung wird die Position des interessierenden Teilbereichs auf dem zweiten Bild durch Entgegennahme einer weiteren Benutzereingabe bestimmt. Dies kann hilfreich sein, wenn eine automatische Erkennung des gewünschten Teilbereichs oder Punktes erschwert ist oder ein falsches Ergebnis bringt.

Nach einer weiteren Ausgestaltung der Erfindung umfasst die weitere Positionierung des Aufnahmesystems eine Positionierung des interessierenden Teilbereichs in das Zentrum des Röntgendetektors. Bei vorliegendem SOD kann ganz einfach berechnet werden, welche weitere Verschiebung das Aufnahmesystem benötigt, um den Teilbereich in das Zentrum zu bewegen und diese Verschiebung kann anschließend umgesetzt werden.

Nach einer weiteren Ausgestaltung der Erfindung wird das Verfahren wiederholt, sobald eine Änderung hinsichtlich eines weiteren interessierenden Teilbereichs erfolgt. Da ein anderer Teilbereich innerhalb des Patienten, welcher sich nicht in derselben Ebene befindet, den SOD verändert, ist es in diesem Fall notwendig, das Verfahren zu wiederholen, um wieder ein exaktes automatisches Positionieren des Aufnahmesystems gewährleisten zu können. Dasselbe gilt auch dafür, wenn der Patient auf der Patientenliege einen Hub in Bezug auf die Ebene erfährt.

Die Erfindung umfasst außerdem ein Röntgenbildgebungssystem zur Durchführung eines erfindungsgemäßen Verfahrens, aufweisend ein Aufnahmesystem mit einer Röntgenquelle und einem Röntgendetektor, eine Systemsteuerung zur automatischen Ansteuerung des Röntgenbildgebungssystems, eine Bildbearbeitungseinheit zur Bearbeitung von Röntgenbildern, eine Eingabeeinheit zur Entgegennahme von Benutzereingaben, einer Berechnungseinheit, eine Bildausgabeeinheit und eine Speichereinheit. Das Röntgenbildgebungssystem kann insbesondere von einem mobilen C-Bogen-Röntgensystem gebildet werden.

Die Erfindung sowie weitere vorteilhafte Ausgestaltungen gemäß Merkmalen der Unteransprüche werden im Folgenden anhand schematisch dargestellter Ausführungsbeispiele in der Zeichnung näher erläutert, ohne dass dadurch eine Beschränkung der Erfindung auf diese Ausführungsbeispiele erfolgt. Es zeigen:

FIG 1    eine Ansicht eines erfindungsgemäßen Röntgenbildgebungssystems;

FIG 2    eine Ansicht der geometrischen Zusammenhänge zur Bestimmung eines SOD;

FIG 3    eine Ansicht der beiden Röntgenbilder mit darauf abgebildetem interessierenden Teilbereich; und

FIG 4    ein Ablauf des erfindungsgemäßen Verfahrens.

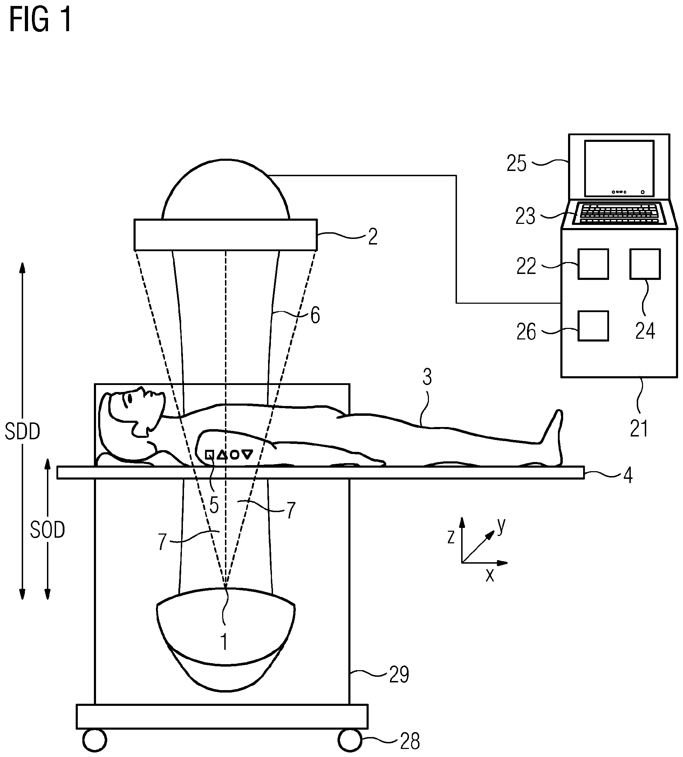

In der FIG 1 ist ein erfindungsgemäßes Röntgenbildgebungssystem 27 mit einem Aufnahmesystem in Form eines C-Bogens 6 mit einer Röntgenquelle 1 und einem Röntgendetektor 2 gezeigt. Das Röntgenbildgebungssystem weist außerdem eine Systemsteuerung 21 auf, welche die Funktionen des Röntgenbildgebungssystems ansteuert, also z.B. die Aussendung von Röntgenstrahlung, die Bilderfassung usw. Außerdem sind eine Bildberabeitungseinheit 22 zur Bearbeitung von Röntgenbildern, eine Berechnungseinheit 24 zur Berechnung von Daten, eine Speichereinheit zur Speicherung von Daten sowie eine Anzeigeeinheit 25 in Form eines Monitors und eine Eingabeeinheit 23 in Form einer Tastatur und Maus vorhanden. Alternativ oder zusätzlich können auch Touchscreens oder Smart Devices für eine Anzeige und Eingabe vorhanden sein.

Der C-Bogen 6 ist z.B. an einem Gerätewagen 29 bewegbar befestigt und kann z.B. orbital und horizontal verstellt bzw. bewegt werden. Der Gerätewagen 29 mit dem C-Bogen 6 kann manuell oder automatisch mittels Rollen 28 verfahren werden.

Auf einer Patientenliege 4 liegt als Untersuchungsobjekt ein Patient 3, welcher von der Röntgenstrahlung in Form eines Kegelstrahls 7 durchleuchtet wird. Der kürzest mögliche Abstand zwischen der Röntgenquelle 1 und dem Röntgendetektor 2, im Allgemeinen das Lot von der Röntgenquelle 1 auf den Röntgendetektor 2, wird als Röntgenquelle-Röntgendetektor-Abstand (SDD) bezeichnet. Für eine automatische Positionierung eines Teilbereichs 5, z.B. eines Wirbelkörpers, des Patienten 3 ist das Röntgenbildgebungssystem 27 zur Ausführung des erfindungsgemäßen Verfahrens ausgebildet. Im Rahmen des Verfahrens wird der genaue Abstand zwischen der Röntgenquelle und dem interessierenden Teilbereich (SOD) bestimmt und für die Positionierung verwendet. Zur genaueren Darstellung der geometrischen Zusammenhänge ist ein Koordinatensystem xyz gezeigt, wobei die z-Achse parallel zum SDD ist und die x-Achse und die y-Achse eine Ebene parallel zum Röntgendetektor aufspannen. Die Problematik, welche durch das Verfahren gelöst wird, besteht im Allgemeinen darin, dass der Abstand zwischen der Röntgenquelle und dem Patienten auf einfache Weise messbar ist, jedoch der Abstand zu einem Teilbereich des Patienten (SOD), für den nicht so einfach erkennbar ist, in welcher Ebene des Patienten er sich befindet, sehr schwierig ist.

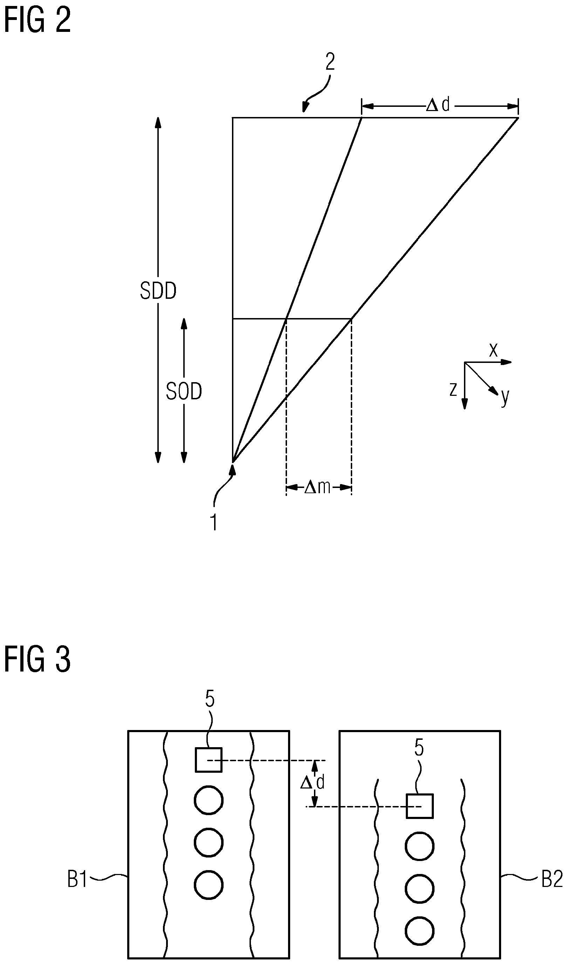

In der FIG 2 sind die geometrischen Zusammenhänge nochmal vergrößert dargestellt. Mittels des Strahlensatzes kann aus dem SDD, einem Verschiebeabstand ∆m und einem Pixelabstand ∆d der SOD bestimmt werden:

$$SOD = SDD \cdot \frac{\Delta m}{\Delta d}$$

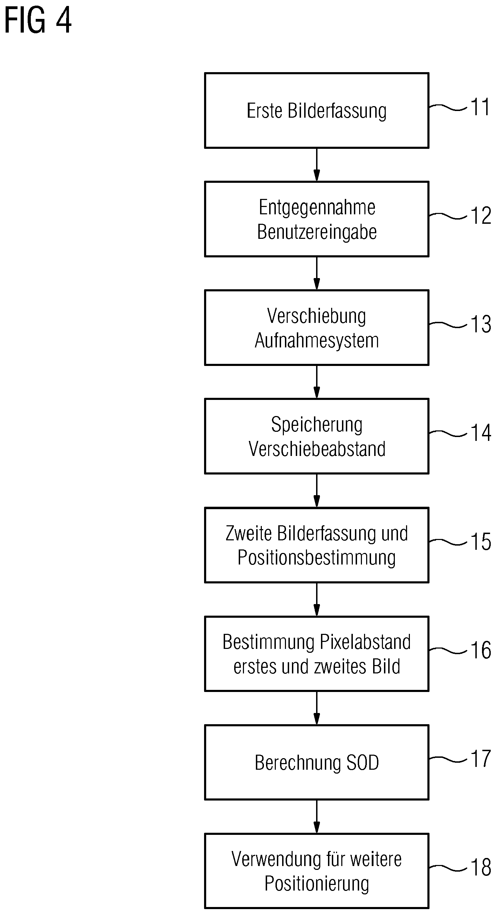

Im Folgenden wird der Ablauf des Verfahrens anhand von FIG 4 erläutert, wobei zunächst eine Kalibrierung hinsichtlich des SOD erfolgt und anschließend auf der Basis der Kalibrierung eine Positionierung erfolgen kann:

In einem ersten Schritt 11 wird ein erstes Röntgenbild B1 (siehe auch FIG 3) des Untersuchungsobjekts erfasst und bevorzugt anschließend auf einer Anzeigeeinheit 25 dargestellt. Anschließend wird in einem zweiten Schritt 12 eine Benutzereingabe hinsichtlich eines interessierenden Teilbereichs 5 auf dem Röntgenbild B1 entgegengenommen und gespeichert. Hierfür kann der Nutzer z.B. mittels der Eingabeeinheit 23 den Teilbereich oder einen Punkt darauf markieren. Besonders vorteilhaft ist hierfür die Nutzung eines Touchscreens, auf dem der Nutzer direkt auf dem angezeigten Röntgenbild die entsprechenden Positionen markiert.

In einem dritten Schritt 13 wird das Aufnahmesystem automatisch derart verstellt, dass eine hubfreie Verschiebung um einen Verschiebeabstand ∆m zwischen Aufnahmesystem und Untersuchungsobjekt in einer Ebene parallel zum Röntgendetektor resultiert. Die Verstellung kann z.B. von einer einfachen Translation des Röntgenbildgebungssystems oder Bewegung des C-Bogens in x-Richtung und/oder y-Richtung, also entlang des Patienten, gebildet werden. Derartige Verstellungen sind bekannt und können auf einfache Weise angesteuert werden. So kann z.B. die Verschiebung derart automatisch angesteuert werden, dass die Systemsteuerung zunächst einen geschätzten SOD, einen mittels Messungen wie ToF, Lasertriangulation oder Ultraschall ermittelten SOD oder einen Durchschnitts-SOD annimmt und auf der Basis des angenäherten SOD versucht, den interessierenden Teilbereich bezüglich des Röntgendetektors zu zentrieren. Es kann auch z.B. ein voreingestellter Standardwert für eine Verschiebung verwendet werden.

In einem vierten Schritt 14 wird der absolute Verschiebeabstand gespeichert, dieser kann z.B. aus Encodern in einem Gelenk des Aufnahmesystems oder den Rollen/Rädern des Gerätewagens entnommen oder von der Systemsteuerung abgefragt werden.

In einem fünften Schritt 15 wird anschließend ein zweites Röntgenbild B2 aufgenommen und auf diesem zweiten Röntgenbild B2 die Position des interessierenden Teilbereichs bzw. Punkts bestimmt. Die Positionsbestimmung kann z.B. mittels eines Objekterkennungsalgorithmus oder eines Algorithmus zur Bilderkennung oder Kantendetektion durchgeführt werden. Alternativ oder zusätzlich kann eine weitere Benutzereingabe hinsichtlich des Teilbereichs 5 entgegengenommen werden. Anschließend wird in einem sechsten Schritt 16 die Position des Teilbereichs 5 auf dem ersten Röntgenbild B1 und dem zweiten Röntgenbild B2 verglichen und der absolute Pixelabstand ∆d in Richtung der Verschiebung bestimmt, z.B. mittels der Bildbearbeitungseinheit in Kombination mit der Berechnungseinheit. Dies ist z.B. in der FIG 3 schematisch gezeigt. Aus den nun bekannten Werten für den SSD, den Pixelabstand ∆d und den Verschiebeabstand ∆m wird in einem siebten Schritt 17 auf einfache Weise mittels des Strahlensatzes der SOD berechnet, z.B. durch die Berechnungseinheit. Anschließend kann der SOD in einem achten Schritt 18 für eine exakte Positionierung des Teilbereichs verwendet werden, z.B. für eine Positionierung des Teilbereichs im Zentrum des Röntgendetektors bzw. eines Röntgenbildes. Dafür wird mittels des SOD berechnet, wohin das Aufnahmesystem verstellt werden muss, um den Teilbereich zu zentrieren und die Verstellung mittels der Systemsteuerung umgesetzt.

Für den Nutzer stellt sich der Workflow durch das Verfahren nun folgendermaßen dar: Der Patient wird auf der Patientenliege positioniert, das Aufnahmesystem wird ebenfalls positioniert und das Verfahren wird gestartet. Dem Nutzer wird dann z.B. auf einem Touchscreen das erste Röntgenbild angezeigt und er markiert den interessierenden Teilbereich. Die weiteren Schritte verlaufen automatisch, so dass am Ende des Verfahrens der vom Nutzer gewählte, interessierende Teilbereich zentriert bezüglich des Röntgendetektors positioniert ist.

Die Erfindung lässt sich in folgender Weise kurz zusammenfassen: Die Erfindung umfasst ein Verfahren zur automatischen Positionierung eines Aufnahmesystems mit einem Röntgendetektor und einer Röntgenquelle bezüglich eines Teilbereichs eines Untersuchungsobjekts, mit den folgenden Schritten: Erste Bilderfassung des Untersuchungsobjekts, Entgegennahme einer Benutzereingabe hinsichtlich eines interessierenden Teilbereichs des Untersuchungsobjekts auf dem ersten Bild und Speicherung der Position des Teilbereichs; Automatische Verstellung des Aufnahmesystems derart, dass eine hubfreie Verschiebung zwischen Aufnahmesystem und Untersuchungsobjekt in einer Ebene parallel zum Röntgendetektor resultiert, Speicherung des Verschiebeabstands ($\Delta$m), Zweite Bilderfassung des Untersuchungsobjekts und Bestimmung der Position des interessierenden Teilbereichs auf dem zweiten Bild, Bestimmung des relativen Pixelabstands ($\Delta$d) zwischen der Position des interessierenden Teilbereichs auf dem ersten und auf dem zweiten Bild; Berechnung des SOD (Röntgenquelle-Untersuchungsobjekt-Abstands) aus dem Verschiebeabstand, dem relativen Pixelabstand und dem bekannten SDD (Röntgenquelle-Röntgendetektor-Abstand), und Verwendung des SOD für eine automatische Positionierung des Aufnahmesystems. Der SOD wird nach der Formel $SOD = SDD \cdot \frac{\Delta m}{\Delta d}$ berechnet.

**Patentansprüche**

1. Verfahren zur automatischen Positionierung eines Aufnahmesystems (1; 2; 6) mit einem Röntgendetektor (2) und einer Röntgenquelle (1) bezüglich eines Teilbereichs (5) eines Untersuchungsobjekts, mit den folgenden Schritten:

    • Erste Bilderfassung des Untersuchungsobjekts,
    • Entgegennahme einer Benutzereingabe hinsichtlich eines interessierenden Teilbereichs (5) des Untersuchungsobjekts auf dem ersten Bild und Speicherung der Position des Teilbereichs;
    • Automatische Verstellung des Aufnahmesystems (1; 2; 6) derart, dass eine insbesondere hubfreie Verschiebung zwischen Aufnahmesystem (1; 2; 6) und Untersuchungsobjekt in einer Ebene parallel zum Röntgendetektor resultiert,
    • Speicherung des Verschiebeabstands ($\Delta$m),
    • Zweite Bilderfassung des Untersuchungsobjekts und Bestimmung der Position des interessierenden Teilbereichs (5) auf dem zweiten Bild,
    • Bestimmung des relativen Pixelabstands ($\Delta$d) zwischen der Position des interessierenden Teilbereichs (5) auf dem ersten und auf dem zweiten Bild;
    • Berechnung des SOD (Röntgenquelle-Untersuchungsobjekt-Abstands) aus dem Verschiebeabstand ($\Delta$m), dem relativen Pixelabstand ($\Delta$d) und dem bekannten SDD (Röntgenquelle-Röntgendetektor-Abstand), und
    • Verwendung des SOD für eine automatische Positionierung des Aufnahmesystems (1; 2; 6).

2. Verfahren nach Anspruch 1, wobei der SOD nach der Formel $SOD = SDD \cdot \frac{\Delta m}{\Delta d}$ berechnet wird.

3. Verfahren nach einem der vorangehenden Ansprüche, wobei die weitere Positionierung des Aufnahmesystems (1; 2; 6) eine Positionierung des interessierenden Teilbereichs (5) in das Zentrum des Röntgendetektors (2) umfasst.

4. Verfahren nach einem der vorangehenden Ansprüche, wobei die Position des interessierenden Teilbereichs (5) auf dem zweiten Bild automatisch mittels Bilderkennung oder Kantendetektion oder Objekterkennung ermittelt wird.

5. Verfahren nach einem der vorangehenden Ansprüche, wobei das Verfahren wiederholt wird, sobald eine Änderung hinsichtlich eines weiteren interessierenden Teilbereichs erfolgt.

6. Verfahren nach einem der vorangehenden Ansprüche, wobei die Position des interessierenden Teilbereichs (5) auf dem zweiten Bild durch Entgegennahme einer weiteren Benutzereingabe bestimmt wird.

7. Röntgenbildgebungssystem zur Durchführung eines Verfahrens nach einem der Ansprüche 1 bis 6, aufweisend ein Aufnahmesystem (1; 2; 6) mit einer Röntgenquelle (1) und einem Röntgendetektor (2), eine Systemsteuerung (21) zur automatischen Ansteuerung des Röntgenbildgebungssystems, eine Bildbearbeitungseinheit (22) zur Bearbeitung von Röntgenbildern, eine Eingabeeinheit (23) zur Entgegennahme von Benutzereingaben, einer Berechnungseinheit (24), eine Bildausgabeeinheit (25) und eine Speichereinheit (26).

8. Röntgenbildgebungssystem nach Anspruch 7, welches von einem mobilen C-Bogen-Röntgensystem gebildet wird.

FIG 1

# FIG 2

# FIG 3

# FIG 4

```
┌─────────────────────┐
│  Erste Bilderfassung │ ───── 11
└─────────────────────┘
           │
           ▼
┌─────────────────────┐
│     Entgegennahme    │ ───── 12
│    Benutzereingabe   │
└─────────────────────┘
           │
           ▼
┌─────────────────────┐
│      Verschiebung    │ ───── 13
│    Aufnahmesystem    │
└─────────────────────┘
           │
           ▼
┌─────────────────────┐
│      Speicherung     │ ───── 14
│   Verschiebeabstand  │
└─────────────────────┘
           │
           ▼
┌─────────────────────┐
│ Zweite Bilderfassung und │ ─── 15
│   Positionsbestimmung │
└─────────────────────┘
           │
           ▼
┌─────────────────────┐
│ Bestimmung Pixelabstand │ ─── 16
│  erstes und zweites Bild │
└─────────────────────┘
           │
           ▼
┌─────────────────────┐
│    Berechnung SOD    │ ───── 17
└─────────────────────┘
           │
           ▼
┌─────────────────────┐
│  Verwendung für weitere │ ── 18
│     Positionierung   │
└─────────────────────┘
```

Europäisches
Patentamt

European
Patent Office

Office européen
des brevets

**EUROPÄISCHER RECHERCHENBERICHT**

Nummer der Anmeldung

EP 18 16 1773

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| X | JP 2015 195970 A (TOSHIBA CORP; TOSHIBA MEDICAL SYS CORP)<br>9. November 2015 (2015-11-09)<br>* Absatz [0009] - Absatz [0085]; Abbildungen *<br>----- | 1-8 | INV.<br>A61B6/00 |
| A | DE 10 2012 205238 A1 (SIEMENS AG [DE])<br>2. Oktober 2013 (2013-10-02)<br>* Absatz [0034] - Absatz [0048]; Abbildungen *<br>----- | 1-8 | |
| A | DE 10 2006 055133 A1 (SIEMENS AG [DE])<br>5. Juni 2008 (2008-06-05)<br>* Absatz [0053] - Absatz [0062] *<br>* Absatz [0080] - Absatz [0101]; Abbildungen *<br>----- | 1-8 | |
| A | US 2010/329416 A1 (TSUJII OSAMU [JP])<br>30. Dezember 2010 (2010-12-30)<br>* Absatz [0026] - Absatz [0028]; Abbildungen *<br>----- | 1-8 | RECHERCHIERTE SACHGEBIETE (IPC)<br><br>A61B |

Der vorliegende Recherchenbericht wurde für alle Patentansprüche erstellt

| Recherchenort | Abschlußdatum der Recherche | Prüfer |
|---|---|---|
| München | 19. September 2018 | Strubel, Christine |

KATEGORIE DER GENANNTEN DOKUMENTE

X : von besonderer Bedeutung allein betrachtet
Y : von besonderer Bedeutung in Verbindung mit einer
    anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder
    nach dem Anmeldedatum veröffentlicht worden ist
D : in der Anmeldung angeführtes Dokument
L : aus anderen Gründen angeführtes Dokument
    ...........................................................................
& : Mitglied der gleichen Patentfamilie, übereinstimmendes
    Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 18 16 1773

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

19-09-2018

| Im Recherchenbericht angeführtes Patentdokument | Datum der Veröffentlichung | Mitglied(er) der Patentfamilie | Datum der Veröffentlichung |
|---|---|---|---|
| JP 2015195970 A | 09-11-2015 | KEINE | |
| DE 102012205238 A1 | 02-10-2013 | KEINE | |
| DE 102006055133 A1 | 05-06-2008 | KEINE | |
| US 2010329416 A1 | 30-12-2010 | CN 102365051 A | 29-02-2012 |
| | | JP 5460106 B2 | 02-04-2014 |
| | | JP 2010240106 A | 28-10-2010 |
| | | US 2010329416 A1 | 30-12-2010 |
| | | US 2011188628 A1 | 04-08-2011 |
| | | US 2013003920 A1 | 03-01-2013 |
| | | WO 2010113415 A1 | 07-10-2010 |

EPO FORM P0461

Für nähere Einzelheiten zu diesem Anhang : siehe Amtsblatt des Europäischen Patentamts, Nr.12/82